(19) **Europäisches Patentamt / European Patent Office / Office européen des brevets**

(11) **EP 3 435 074 A1**

(12) **EUROPEAN PATENT APPLICATION**
published in accordance with Art. 153(4) EPC

(43) Date of publication:
**30.01.2019 Bulletin 2019/05**

(21) Application number: **17769648.1**

(22) Date of filing: **27.01.2017**

(51) Int Cl.:
**G01N 27/04** $^{(2006.01)}$

(86) International application number:
**PCT/JP2017/002880**

(87) International publication number:
**WO 2017/163601 (28.09.2017 Gazette 2017/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **22.03.2016 JP 2016056568**

(71) Applicant: **Sintokogio, Ltd.
Nagoya-shi, Aichi 450-6424 (JP)**

(72) Inventor: **AOKI, Yukinori
Kurate-gun
Fukuoka 807-1306 (JP)**

(74) Representative: **Beyer, Andreas
Wuesthoff & Wuesthoff
Patentanwälte PartG mbB
Schweigerstrasse 2
81541 München (DE)**

(54) **SAND CONTAMINATION DEGREE EVALUATING METHOD AND SAND CONTAMINATION DEGREE EVALUATING DEVICE FOR FOUNDRY SAND**

(57) A method and an apparatus for evaluating the degree of contamination of foundry sand are provided that can measure the electrical conductivity of the foundry sand to evaluate the degree of contamination based on the measured electrical conductivity. The method for evaluating the degree of contamination of the foundry sand comprises the steps of preparing a standard sample (1, 101) and a comparative sample (201) of the foundry sand, measuring the electrical conductivity of the standard and comparative samples after a wetting treatment of them has been carried out, and evaluating the degree of contamination based on the measured electrical conductivity.

Fig. 1

## Description

Technical Field

The present invention relates to a method and an apparatus for evaluating the degree of contamination of foundry sand, wherein electrical conductivity (EC) is used as an index for the evaluation of the degree of the contamination of the foundry sand.

Background Art

Conventionally, in the agricultural field, a way to measure the electrical conductivity of soil by causing an electrode to directly contact the soil has been known (for example, see Patent Literature 1).

However, the method disclosed in Patent Literature 1 assumes that soil contains a certain amount of moisture. Thus, that method cannot be applied to foundry sand, which is normally dry. Further, since the foundry sand is normally dry and thus is an electrical insulator, no technological conception to measure the electrical conductivity of foundry sand has existed. Thus evaluating the degree of the contamination of foundry sand has been very difficult.

The present invention has been conceived in consideration of the above-mentioned problems. Its objectives are to provide a method and an apparatus for evaluating the degree of contamination of foundry sand, wherein the electrical conductivity of foundry sand can be measured, and wherein the degree of contamination of the foundry sand can be evaluated based on the measured electrical conductivity.

Prior-art Publication

Patent Literature

[Patent Literature 1]
Japanese Patent Laid-open Publication No. 2001-215203

Disclosure of Invention

To achieve the above-mentioned objectives, a method for evaluating a degree of contamination of foundry sand of the present invention is a method to evaluate a degree of contamination of foundry sand by measuring electrical conductivity of the foundry sand. It comprises a step of preparing a standard sample and a comparative sample of the foundry sand. It also comprises a step of measuring electrical conductivity of the standard sample and the comparative sample after a wetting treatment of the standard sample and the comparative sample has been carried out. It also comprises a step of evaluating the degree of contamination based on the measured electrical conductivity of the standard sample and the comparative sample.

By the method for evaluating the degree of the contamination of the foundry sand of the present invention, the wetting treatment of the standard sample and the comparative sample are carried out after a drying treatment, and then the electrical conductivity of both samples is measured.

By the method for evaluating the degree of the contamination of the foundry sand of the present invention, the electrical conductivity of the standard sample and the comparative sample is measured after the drying treatment and the wetting treatment are alternately repeated multiple times.

The method for evaluating the degree of the contamination of the foundry sand of the present invention also comprises a step of measuring temperatures of the standard sample and the comparative sample.

The method for evaluating the degree of the contamination of the foundry sand of the present invention also comprises a step of calculating a difference in temperatures between the standard sample and the comparative sample. It also comprises a step of modifying the measured electrical conductivity of the standard sample and the comparative sample by increasing or decreasing the measured electrical conductivity by a value of electrical conductivity that corresponds to the difference in temperatures.

By the method for evaluating the degree of the contamination of the foundry sand of the present invention, data for evaluating the degree of contamination are used as a reference that is used to control a pressure caused by a roller of an apparatus for reclaiming foundry sand.

By the method for evaluating the degree of the contamination of the foundry sand of the present invention a plurality of the standard samples are used.

To achieve the above-mentioned objectives, an apparatus for evaluating a degree of contamination of foundry sand of the present invention is an apparatus for evaluating a degree of contamination of foundry sand by measuring electrical conductivity of the foundry sand. It comprises a container of a standard sample that holds a standard sample of the foundry sand. It also comprises a means for measuring electrical conductivity of a standard sample that measures the electrical conductivity of the standard sample that is held in the container of the standard sample. It also comprises a means for wetting a standard sample that wets the standard sample that is held in the container of the standard sample. It also comprises a container of a comparative sample that holds a comparative sample of the foundry sand. It also comprises a means for measuring electrical conductivity of a comparative sample that measures the electrical conductivity of the comparative sample that is held in the container of the comparative sample. It also comprises a means for wetting a comparative sample that wets the comparative sample that is held in the container of the comparative sample.

The apparatus for evaluating a degree of contamination of the foundry sand of the present invention also comprises a means for drying a standard sample that dries the standard sample that is held in the container of the standard sample. It also comprises a means for drying a comparative sample that dries the comparative sample that is held in the container of the comparative sample.

The apparatus for evaluating a degree of contamination of the foundry sand of the present invention also comprises a means for measuring a temperature of a standard sample that measures a temperature of the standard sample that is held in the container of the standard sample. It also comprises a means for measuring a temperature of a comparative sample that measures a temperature of the comparative sample that is held in the container of the comparative sample.

The present invention is a method for evaluating the degree of contamination of foundry sand by measuring the electrical conductivity of the foundry sand. It comprises a step of preparing a standard sample and a comparative sample of the foundry sand, a step of measuring the electrical conductivity of the standard sample and the comparative sample after a wetting treatment of the standard sample and the comparative sample, and a step of evaluating the degree of contamination based on the measured electrical conductivity of the standard sample and the comparative sample. Thus various advantageous effects can be obtained, such as that the electrical conductivity of foundry sand can be measured, and that the degree of contamination can be evaluated based on the measured electrical conductivity.

The basic Japanese patent application, No. 2016-056568, filed March 22, 2016, is hereby incorporated by reference in its entirety in the present application.

The present invention will become more fully understood from the detailed description given below. However, that description and the specific embodiments are only illustrations of the desired embodiments of the present invention, and so are given only for an explanation. Various possible changes and modifications will be apparent to those of ordinary skill in the art on the basis of the detailed description.

The applicant has no intention to dedicate to the public any disclosed embodiment. Among the disclosed changes and modifications, those which may not literally fall within the scope of the present claims constitute, therefore, under the doctrine of equivalents, a part of the present invention.

The use of the articles "a," "an," and "the" and similar referents in the specification and claims are to be construed to cover both the singular and the plural form of a noun, unless otherwise indicated herein or clearly contradicted by the context. The use of any and all examples, or exemplary language (e.g., "such as") provided herein is intended merely to better illuminate the invention, and so does not limit the scope of the invention, unless otherwise stated.

Brief Description of Drawings

[Fig. 1]

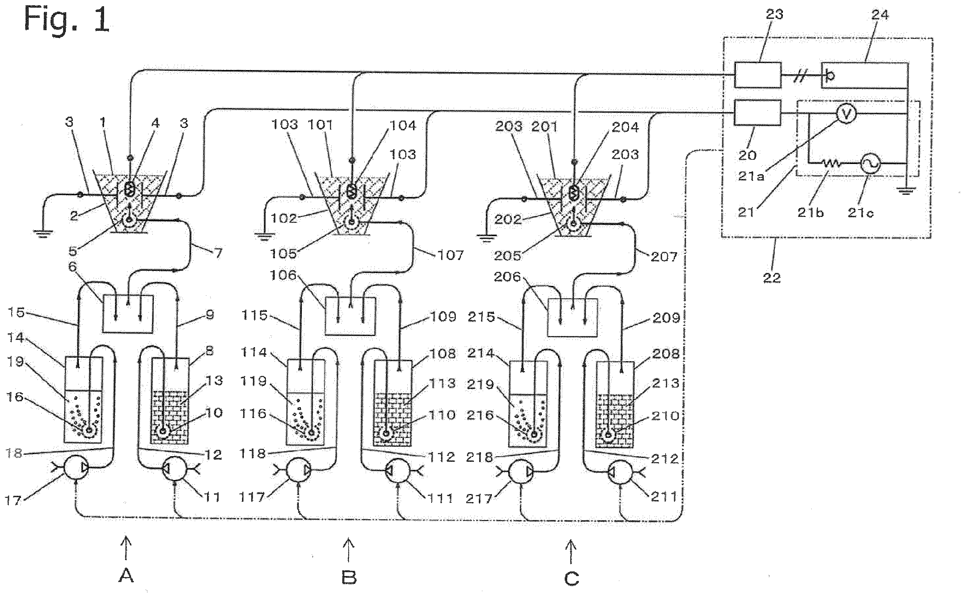

Fig. 1 is a schematic block diagram that shows an embodiment of the present invention.

Mode for Carrying Out the Invention

Below, with reference to the drawing, the embodiments of the present invention are discussed in detail. In the present embodiments two kinds of a standard sample and one kind of a comparative sample are used. The standard samples are foundry sand (two samples), whose properties to be used for evaluating the degree of contamination are known. The comparative sample is foundry sand (a sample) that is to be compared with the standard samples. Thus the degree of contamination of the comparative sample is to be evaluated. The degree of contamination indicates the degree to which, for example, resin, hardening agents, or auxiliary agents, which are added to foundry sand, or casting scrap, are contained. The resin may be an alkaline phenolic resin, a furan resin, a phenolic resin, a polyol resin, etc. The hardening agents may be an organic acid, polyisocyanate, an organic ester, etc. The auxiliary agents may be a metallic soap, a surfactant, etc. However, they are not limited to these substances.

At station A for the first standard sample 1, as in Fig. 1, the first standard sample 1 is held in a container 2 of the first standard sample. In the container 2 of the first standard sample, electrodes 3 for measuring the electrical conductivity of the first standard sample 1, which are a means for measuring the electrical conductivity of the first standard sample, are inserted through both sides of the container 2, and are placed in it.

In the container 2 of the first standard sample, a temperature sensor 4 for measuring the temperature of the first standard sample 1, which is a means for measuring the temperature of the first standard sample, is inserted from above and is placed in it. An aerator 5 is provided below both the electrodes 3 and the temperature sensor 4, in the container 2 of the first standard sample.

An intermediate container 6 is provided away from the container 2 of the first standard sample. The aerator 5 and the intermediate container 6 are connected by a piping 7. A means 8 for drying the first standard sample that dries the first standard sample 1 that is held in the container 2 of the first standard sample is provided away from the intermediate container 6. The intermediate container 6 and the means 8 for drying the first standard sample are connected by a piping 9.

An aerator 10 is provided in the means 8 for drying the first standard sample. An air pump 11 is provided away from the means 8 for drying the first standard sample. The aerator 10 and the air pump 11 are connected by a piping 12. Within the means 8 for drying the first standard sample a specific amount of granulated silica gel 13 is held.

A means 14 for drying the first standard sample that dries the first standard sample 1 that is held in the container 2 of the first standard sample is provided away from the means 8 for drying the first standard sample. The intermediate container 6 and the means 14 for drying the first standard sample are connected by the piping 15. An aerator 16 is provided in the means 14 for drying the first standard sample. An air pump 17 is provided away from the means 14 for drying the first standard sample. The aerator 16 and the air pump 17 are connected by a piping 18. A specific amount of water 19 is held in the means 14 for drying the first standard sample.

The configuration of station A for the first standard sample 1 is discussed above. Since the configurations of a station B for the second standard sample 101 and a station C for the comparative sample 201 are the same as the configuration of station A for the first standard sample 1, the explanation of them is omitted. Incidentally, the numbers in the 100 series are used for station B for the second standard sample 101, and the numbers in the 200 series are used for station C for the comparative sample 201.

One end of each of the electrodes 3, 103, and 203 of stations A, B, and C is grounded, and the other end is electrically connected to a circuit 21 for measuring the electrical conductivity through a switching circuit 20. The switching circuit 20 and the circuit 21 for measuring the electrical conductivity are incorporated in a controller 22. The switching circuit 20 switches on whichever of the electrodes 3, 103, and 203 is used for the measurement. In the circuit 21 for measuring the electrical conductivity the reference numbers "21a," "21b," and "21c" denote a voltmeter, a resistance, and an alternating-current source, respectively.

The temperature sensors 4, 104, and 204 of stations A, B, and C are electrically connected to a circuit 24 for measuring the temperature through a switching circuit 23. The switching circuit 23 and the circuit 24 for measuring the temperature are incorporated in the controller 22. The switching circuit 23 switches on whichever of the temperature sensors 4, 104, and 204 is used for the measurement. The air pumps 11, 17, 111, 117, 211, and 217 of stations A, B, and C are electrically connected to the controller 22.

The operations of the apparatus for evaluating the degree of the contamination of the foundry sand thus configured is now discussed. First, two kinds of the standard sample of, and one kind of the comparative sample of, self-hardening foundry sand, are prepared.

Sample

Aggregate for the foundry sand:

Product name: "Lunamos" (Kao Corporation, Japan. Artificial sand)

Additive resin (self-hardening process):

Alkaline phenolic resin (water-soluble)

Note: Three kinds of samples, which each have a different LOI (Loss of Ignition), are prepared. They are:

first standard sample 1: the LOI is 0.64% (high quality)
second standard sample 101: the LOI is 1.06% (low quality)
comparative sample 201: the LOI is 0.88% (medium quality)

A specific amount of the first standard sample 1 is supplied to the container 2 of the first standard sample at station A. A specific amount of the second standard sample 101 is supplied to the second container 102 of the standard sample at station B. A specific amount of the comparative sample 201 is supplied to the container 202 of the comparative sample at station C. Incidentally, these samples may be supplied manually or mechanically.

Subsequent operations for stations A, B, and C are the same. Thus, only the operations for station A are discussed. At station A, first, a counter (not shown) for steps increases the number of steps. The counter for steps counts the number of drying treatments and wetting treatments of the first standard sample 1, which are alternately repeated, and which are discussed later. When both the drying treatment and the wetting treatment are carried out once, one is added to the number of steps that have been counted by the counter for steps. Next, a timer (not shown) starts to measure the periods of time. The timer stores data on the period for a drying treatment and the period for a wetting treatment.

Next, measuring the electrical conductivity of the first standard sample 1 by means of the electrodes 3 is started. The electrical conductivity is the reciprocal of a resistance, which resistance is measured by the circuit 21 for measuring the electrical conductivity. Measuring the temperature of the first standard sample 1 by means of the temperature sensor 4 is started. Incidentally, the measurements of the electrical conductivity and the temperature are continually repeated at predetermined intervals of time.

Next, the drying treatment and the wetting treatment of the first standard sample 1 are carried out. Below, these operations are discussed in detail. The air pump 11 is activated so that air is blown out of the aerator 10. The air that has been blown out becomes dry while it passes through the gaps in the granulated silica gel 13 that is held in the means 8 for drying the first standard sample. It is supplied to the intermediate container 6 through the piping 9. The dry air that has been supplied to the intermediate container 6 is supplied to the aerator 5 through the piping 7. The drying treatment of the first standard sample 1 is carried out by means of the dry air that is blown out of the aerator 5. The drying treatment is carried out for a specific period of time (the data on the period is stored by the timer).

Then, the air pump 11 is deactivated. The operation is switched from the drying treatment to the wetting treatment. In the wetting treatment, the air pump 17 is activated so that the air is blown out of the aerator 16. The air that has been blown out becomes wet while it passes through water 19 that is held in the means 14 for wetting the first standard sample. It is supplied to the intermediate container 6 through the piping 15. The wet air that has been supplied to the intermediate container 6 is supplied to the aerator 5 through the piping 7. The wetting treatment of the first standard sample 1 is carried out by means of the wet air that is blown out of the aerator 5. The wetting treatment is carried out for a specific period of time (the data on the period is stored by the timer).

The operations for station A are discussed above. The same operations are carried out for stations B and C. The temperatures of the first standard sample 1, the second standard sample 101, and the comparative sample 201, are measured by means of the temperature sensors 4, 104, and 204, respectively. If any of the following conditions is not fulfilled, the operation returns to the step wherein the counter for steps increases the number, so that the operations after that step are repeated.

(1) The number of steps that are counted by the counter for steps are equal to or above a predetermined number.
(2) The periods of time of the drying treatment and the wetting treatment that are measured by the timer are equal to or longer than a predetermined period.
(3) The differences between the temperatures of any two of the first standard sample 1, the second standard sample 101, and the comparative sample 201, are equal to or under a predetermined value.

Namely, each of the first standard sample 1, the second standard sample 101, and the comparative sample 201, is subject to both the drying treatment and the wetting treatment at least once. In some cases, the drying treatments and the wetting treatments are alternately repeated (multiple times).

If all of the above conditions, namely, (1), (2), and (3), are fulfilled, then the electrical conductivity of each of the first standard sample 1, the second standard sample 101, and the comparative sample 201, is determined. And the drying treatment or the wetting treatment of each of the first standard sample 1, the second standard sample 101, and the comparative sample 201 is stopped.

Next, the degree of contamination is evaluated based on the determined electrical conductivity of each of the first standard sample 1, the second standard sample 101, and the comparative sample 201. This process is discussed regarding the working example, which is discussed below.

Working Example

Below, an embodiment of the present invention is discussed. The samples that are discussed in the above-titled Sample were used for the first standard sample 1, the second standard sample 101, and the comparative sample 201. The counter for steps is set at 3 for both the number of drying treatments and the number of wetting treatments, which are alternately repeated, for stations A, B, and C. The timer sets the period for the drying treatment at 6 minutes and the period for the wetting treatment at 4 minutes.

The electrical conductivity and the temperatures at stations A, B, and C were measured every 12 seconds. The differences in temperatures between any two of the first standard sample 1, the second standard sample 101, and the comparative sample 201, were set at 1 °C.

(1) The number counted by the counter for steps became 3.

(2) The period for the drying treatment became 6 minutes and the period for the wetting treatment became 4 minutes.
(3) The differences in temperatures between any two of the first standard sample 1, the second standard sample 101, and the comparative sample 201, became 0.6 °C.

Since all of the three conditions, (1), (2), and (3), were fulfilled, the electrical conductivity of the first standard sample 1, the second standard sample 101, and the comparative sample 201 were determined. The determined electrical conductivity was 158 $\mu$S (micro Siemens) for the first standard sample 1, 737 $\mu$S for the second standard sample 101, and 343 $\mu$S for the comparative sample 201. These are Ea, Eb, and Ec, which are discussed below.
Based on the determined electrical conductivity, the degree of contamination was evaluated. This procedure is now discussed in detail.

Calculating Coefficient for Evaluation

$$KEc = (Kb-Ka)/(Eb-Ea)\times(Ec-Ea)+Ka \quad (\text{Equation } 1)$$

where Ea: the electrical conductivity of the first standard sample
Eb: the electrical conductivity of the second standard sample
Ec: the electrical conductivity of the comparative sample
Ka: the coefficient for the evaluation of the first standard sample
Kb: the coefficient for the evaluation of the second standard sample
KEc: the coefficient for evaluating the electrical conductivity of the comparative sample

KEc was calculated by using Equation 1 and setting Ka at 0 and Kb at 1.

$$KEc = (1-0)/(737-158)\times(343-158)+0 = 0.32$$

Since Ka of the first standard sample, which is high quality, was set at 0 and Kb of the second standard sample, which is low quality, was set at 1, by using Equation 1 the coefficient for evaluating the electrical conductivity of the comparative sample can be calculated to be a value between 0 and 1 by a linear function.

Evaluating Degree of Contamination_

$$Pc = KEc\times(LOIb-LOIa)+LOIa \quad (\text{Equation } 2)$$

where LOIa: the LOI of the first standard sample
LOIb: the LOI of the second standard sample
KEc: the coefficient for evaluating the electrical conductivity of the comparative sample
Pc: a basis for evaluating the comparative sample

By using Equation 2 the electrical conductivity of the comparative sample was evaluated based on the LOI of the first standard sample and of the second standard sample.

$$Pc = 0.32\times(1.06-0.64)+0.64 = 0.774$$

The basis for evaluating the comparative sample can be calculated based on the LOI of the first standard sample and of the second standard sample, which are known, by using Equation 2.
The basis Pa for evaluating the first standard sample is set to be the same as the LOIa. The basis Pb for evaluating the second standard sample is set to be the same as the LOIb. Therefore, the bases for evaluating the samples became as follows: Pa = 0.64, Pb = 1.06, and Pc = 0.774. The basis Pc for evaluating the comparative sample is 0.774, which is close to 0.88, the actual LOI of the comparative sample. By processing as in the above way, the basis for evaluating the comparative sample can be an approximate value of the LOI and be used for evaluating the degree of contamination. Incidentally, the word "evaluation" used here means to quantitatively measure the degree of contamination.

By the present invention, the data for evaluating the degree of contamination, which are discussed in the above embodiments, can be used as a reference for controlling the pressure applied by the roller in an apparatus for reclaiming foundry sand. The apparatus for reclaiming foundry sand is, for example, an apparatus that strips deposits on the surface of the foundry sand by pressing the layer of the foundry sand by means of the roller. The layer of the foundry sand is formed on the inner surface of the rotary drum, since the foundry sand that is continuously fed into the drum is subject to a centrifugal force. The drum is driven by a motor.

If the data for evaluating the degree of contamination shows that the degree of contamination is higher than a predetermined value, i.e., a targeted value at which the foundry sand is reclaimed by the apparatus for reclaiming foundry sand, then the pressure caused by the roller is raised to increase the power to reclaim the foundry sand. If the data for evaluating the degree of contamination show that the degree of contamination is lower than the predetermined value, then the pressure caused by the roller is reduced, to decrease the power to reclaim the foundry sand. If the data for evaluating the degree of contamination show that the degree of contamination is close to the predetermined value, the power to reclaim the foundry sand remains unchanged. In this way, the pressure applied by the roller of the apparatus for reclaiming foundry sand can be controlled based on the data for evaluating the degree of contamination.

By the present invention, the first and second standard samples 1, 101 and the comparative sample 201, are subject to the drying treatment and then to the wetting treatment. Thereafter the electrical conductivity of them is measured. By this configuration an advantage is obtained wherein a difference in the moisture between the surface layer and the lower layer of any contaminants that adhere to the particles of the foundry sand can be decreased in comparison to that moisture when the samples are subject only to the wetting treatment.

By the present invention, the electrical conductivity of the first and second standard samples 1, 101 and the comparative sample 201 is measured after the drying treatments and the wetting treatments are alternately repeated multiple times. By this configuration an advantage is obtained wherein a difference in the moisture between the surface layer and the lower layer of any contaminants that adhere to the particles of the foundry sand can be decreased.

By the present invention, the temperatures of the first and second standard samples 1, 101 and the comparative sample 201 are measured. By this configuration an advantage is obtained wherein the difference in temperature between any two of the first standard sample 1, the second standard sample 101, and the comparative sample 201 can be calculated by measuring their temperatures so that the electrical conductivity of them is measured under the condition that the differences in temperatures of the samples are as small as possible.

By the present invention, the first standard sample 1, the second standard sample 101, and the comparative sample 201 are subject to the drying treatments and the wetting treatments under the same conditions. Further, the differences in the temperatures of the samples are made as small as possible. Since the electrical conductivity of the samples is measured under such conditions, i.e., circumstances for measurements, they can be measured under conditions that are, as much as possible, the same.

By the present invention, the conditions for determining the electrical conductivity of the first standard sample 1, the second standard sample 101, and the comparative sample 201, may be that the differences in temperatures between any two of them become equal to or below a predetermined value. However, too long a time may pass before the differences become equal to or below the predetermined value. In this case the predetermined value may be increased and the electrical conductivity may be modified based on the temperatures.

To modify the electrical conductivity based on the temperatures, the differences in the temperatures between any two of the samples are calculated when the electrical conductivity is determined. The electrical conductivity that corresponds to the differences in the temperatures is added to, or subtracted from, the measured electrical conductivity that is determined. Thus the electrical conductivity is modified. By in this way modifying the electrical conductivity based on the temperatures, the electrical conductivity of the samples is modified even when the differences in temperatures of the samples are large. Thus, an advantage is obtained wherein the predetermined value for the difference in temperatures can be set to be high so as to shorten the time to determine the electrical conductivity.

By the embodiments of the present invention, two kinds of a standard sample (the first standard sample 1 and the second standard sample 101) are used. However, the present invention is not limited to this. Only one standard sample may be used. However, when a plurality of standard samples are used, the comparative sample is compared with more standard samples. Thus, this is preferable, since evaluating the degree of contamination becomes easy, namely, easily understandable.

By the present invention, the first standard sample 1, the second standard sample 101, and the comparative sample 201, are distinguished based on the LOI (Loss of Ignition). However, the present invention is not limited to this procedure. They may be distinguished based on the amount of acid that is consumed.

By the present invention, the drying treatments and the wetting treatments of the samples are alternately repeated at predetermined times. Because of the wetting treatments, contaminants that adhere to the particles of the foundry sand absorb a certain amount of moisture, so that electricity easily passes through the foundry sand. However, if only the wetting treatment is carried out, a difference in humidity is generated between the surface layer and the lower layer, of the contaminants that adhere to the particles of the foundry sand. If the difference in the thicknesses of the layers is large, the thin layer is first saturated because of the humidity. Thus, the electrical conductivity cannot be accurately measured. By alternately repeating the drying treatments and the wetting treatments, the difference in humidity between the surface layer and the lower layer, of the contaminants that adhere to the particles of the foundry sand, can be reduced.

By the embodiment of the present invention, self-hardening foundry sand that has been used for a self-hardening process with an alkaline phenolic resin (water-soluble) is used as samples (the standard sample and the comparative sample). The sample that can be used for the present invention is not limited to this. Further, for example, self-hardening foundry sand that is used for an inorganic self-hardening process that uses any of liquid glass, cement, and a kind of salt, may be used for the samples. It is not limited to self-hardening foundry sand. Foundry sand that has been used for an inorganic core-making process (core sand) or green sand may be used for the samples.

Below, the main reference numerals and symbols that are used in the detailed description and drawing are listed.

1 the first standard sample
2 the container of the first standard sample
3 the means for measuring the electrical conductivity of the first standard sample
4 the means for measuring the temperature of the first standard sample
5 the aerator
6 the intermediate container
7 the piping
8 the means for drying the first standard sample
9 the piping
10 the aerator
11 the air pump
12 the piping
13 the granulated silica gel
14 the means for drying the first standard sample
15 the piping
16 the aerator
17 the air pump
18 the piping
19 the water
20 the switching circuit
21 the circuit for measuring the electrical conductivity
21a the voltmeter
21b the resistance
21c the alternating-current source
22 the controller
23 the switching circuit
24 the circuit for measuring the temperature
101 the second standard sample
102 the container of the second standard sample
103 the means for measuring the electrical conductivity of the second standard sample
104 the means for measuring the temperature of the second standard sample
105 the aerator
106 the intermediate container
107 the piping
108 the means for drying the second standard sample
109 the piping
110 the aerator
111 the air pump
112 the piping
113 the granulated silica gel
114 the means for drying the second standard sample
115 the piping
116 the aerator

117 the air pump
118 the piping
119 the water
201 the comparative sample
202 the container of the comparative sample
203 the means for measuring the electrical conductivity of the comparative sample
204 the means for measuring the temperature of the comparative sample
205 the aerator
206 the intermediate container
207 the piping
208 the means for drying the comparative sample
209 the piping
210 the aerator
211 the air pump
212 the piping
213 the granulated silica gel
214 the means for drying the comparative sample
215 the piping
216 the aerator
217 the air pump
218 the piping
219 the water
A, B, C the station

**Claims**

1. A method for evaluating a degree of contamination of foundry sand by measuring electrical conductivity of the foundry sand, the method comprising the steps of:

    preparing a standard sample and a comparative sample of the foundry sand;
    measuring electrical conductivity of the standard sample and the comparative sample after a wetting treatment of the standard sample and the comparative sample has been carried out; and
    evaluating the degree of contamination based on the measured electrical conductivity of the standard sample and the comparative sample.

2. The method for evaluating the degree of the contamination of the foundry sand of claim 1, wherein the wetting treatment of the standard sample and the comparative sample are carried out after a drying treatment, and then the electrical conductivity of both samples is measured.

3. The method for evaluating the degree of the contamination of the foundry sand of claim 2, wherein the electrical conductivity of the standard sample and the comparative sample is measured after the drying treatment and the wetting treatment are alternately repeated multiple times.

4. The method for evaluating the degree of the contamination of the foundry sand of any one of claims 1 - 3, further comprising a step of:

    measuring temperatures of the standard sample and the comparative sample.

5. The method for evaluating the degree of the contamination of the foundry sand of claim 4, further comprising steps of:

    calculating a difference in temperatures between the standard sample and the comparative sample; and
    modifying the measured electrical conductivity of the standard sample and the comparative sample by increasing or decreasing the measured electrical conductivity by a value of electrical conductivity that corresponds to the difference in temperatures.

6. The method for evaluating the degree of the contamination of the foundry sand of any one of claims 1 - 3, wherein data for evaluating the degree of contamination are used as a reference that is used to control a pressure applied by a roller of an apparatus for reclaiming foundry sand.

7. The method for evaluating the degree of the contamination of the foundry sand of any one of claims 1 - 3, wherein a plurality of the standard samples are used.

8. An apparatus for evaluating a degree of contamination of foundry sand by measuring electrical conductivity of the foundry sand, the apparatus comprising:

a container of a standard sample that holds a standard sample of the foundry sand;
a means for measuring electrical conductivity of a standard sample that measures the electrical conductivity of the standard sample that is held in the container of the standard sample;
a means for wetting a standard sample that wets the standard sample that is held in the container of the standard sample;
a container of a comparative sample that holds a comparative sample of the foundry sand;
a means for measuring electrical conductivity of a comparative sample that measures the electrical conductivity of the comparative sample that is held in the container of the comparative sample; and
a means for wetting a comparative sample that wets the comparative sample that is held in the container of the comparative sample.

9. The apparatus for evaluating a degree of contamination of the foundry sand of claim 8, further comprising:

a means for drying a standard sample that dries the standard sample that is held in the container of the standard sample; and
a means for drying a comparative sample that dries the comparative sample that is held in the container of the comparative sample.

10. The apparatus for evaluating a degree of contamination of the foundry sand of claim 8 or 9, further comprising:

a means for measuring a temperature of a standard sample that measures a temperature of the standard sample that is held in the container of the standard sample; and
a means for measuring a temperature of a comparative sample that measures a temperature of the comparative sample that is held in the container of the comparative sample.

Fig. 1

## INTERNATIONAL SEARCH REPORT

| International application No. |
|---|
| PCT/JP2017/002880 |

A. CLASSIFICATION OF SUBJECT MATTER
*G01N27/04(2006.01)i*

According to International Patent Classification (IPC) or to both national classification and IPC

B. FIELDS SEARCHED

Minimum documentation searched (classification system followed by classification symbols)
B22C1/00-9/30, G01N27/00-27/10, 27/14-27/24, 33/00-33/46

Documentation searched other than minimum documentation to the extent that such documents are included in the fields searched
Jitsuyo Shinan Koho          1922-1996    Jitsuyo Shinan Toroku Koho    1996-2017
Kokai Jitsuyo Shinan Koho    1971-2017    Toroku Jitsuyo Shinan Koho    1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | CD-ROM of the specification and drawings annexed to the request of Japanese Utility Model Application No. 44552/1991(Laid-open No. 61346/1994) (Hokkaido Sentetsu Imono Kogyo Kumiai), 30 August 1994 (30.08.1994), paragraphs [0001] to [0050]; fig. 1 to 5 (Family: none) | 1-10 |
| A | JP 7-51791 A (Hayasaka Riko Kabushiki Kaisha), 28 February 1995 (28.02.1995), paragraphs [0001] to [0024]; fig. 1 (Family: none) | 1-10 |

☒ Further documents are listed in the continuation of Box C.          ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 March 2017 (15.03.17) | 28 March 2017 (28.03.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2017/002880

C (Continuation). DOCUMENTS CONSIDERED TO BE RELEVANT

| Category* | Citation of document, with indication, where appropriate, of the relevant passages | Relevant to claim No. |
|---|---|---|
| A | JP 58-122148 A (Sinto Kogyo Ltd.), 20 July 1983 (20.07.1983), page 1, lower left column, line 17 to page 3, lower left column, line 11; fig. 1 to 7 (Family: none) | 1-10 |
| A | JP 2014-595 A (Sinto Kogyo Ltd.), 09 January 2014 (09.01.2014), paragraphs [0001] to [0092]; fig. 1 to 9 & US 2015/0096594 A1 paragraphs [0001] to [0130]; fig. 1 to 9 & WO 2013/190888 A1 & DE 112013001502 T5 & MX 2014012288 A & CN 204657398 U | 1-10 |
| A | JP 7-190975 A (Sinto Kogyo Ltd.), 28 July 1995 (28.07.1995), paragraphs [0001] to [0014]; fig. 1 to 6 (Family: none) | 1-10 |
| A | JP 2015-519200 A (Sinto Kogyo Ltd.), 09 July 2015 (09.07.2015), paragraphs [0001] to [0082]; fig. 1 to 4 & US 2015/0114259 A1 paragraphs [0001] to [0105]; fig. 1 to 4 & WO 2013/187341 A1 & DE 112013002952 T5 & TW 201350229 A & CN 104220189 A | 1-10 |

Form PCT/ISA/210 (continuation of second sheet) (January 2015)

**EP 3 435 074 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001215203 A
- JP 2016056568 A